# EUROPEAN PATENT APPLICATION

(11) **EP 2 277 850 A1**
(43) Date of publication of application: **26.01.2011**
(21) Application number: 10184507.1
(22) Date of filing: 19.06.2001
(51) Int. Cl.: C07C 211/35, C07D 207/06, A61K 31/40, A61K 31/13

(54) **1-amino-alkylcyclohexanes as 5-HT3 and neuronal nicotinic receptor antagonists**

(30) Priority: 20.06.2000 US 597102
(62) Divisional of application: 01960342.2
(71) Applicant: Merz Pharma GmbH & Co. KGaA, 60318 Frankfurt (DE)
(72) Inventor: Parsons, Christopher, Graham, Raphael, 61130 Nidderau (DE); Danysz, Wojciech, 61130 Nidderau (DE); Gold, Markus, 64569 Nauheim (DE); Kalvinsh, Ivars, LV-2169 Salaspils (LV); Kauss, Valerjans, LV-1082 Riga (LV); Jirgensons, Aigars, LV-1050 Riga (LV)
(74) Representative: Goddard, Christopher Robert

(57) **Abstract**

Certain 1-aminoalkylcyclohexanes are systematically-active 5HT3 and nicotinic receptor antagonists and are useful in the inhibition of progression of or alleviation of conditions resulting from disturbances of serotoninergic or nicotinergic transmission giving them a wide range of utility in the treatment of CNS-disorders. Pharmaceutical compositions thereof for such purpose and method of making same, as well as a method-of-treating conditions which are alleviated by the employment of a 5HT3 or neuronal nicotinic receptor antagonist.

## Description

### Field of Invention

New uses of 1-amino-alkylcyclohexanes.

### Prior Art

The prior art is represented by our prior USP 6,034,134 of March 7, 2000 and our published application WO 99/01416, PCT/EP98/04026, and Parsons et al. Neuropharmacology 38, 85-108 (1999), wherein the active compounds utilized according to the present invention are disclosed and disclosed to be NMDA receptor antagonists and anticonvulsants.

### The Present Invention

The present invention is directed to a new use of 1-amino-alkylcyclohexane compounds selected from the group consisting of those of the formula 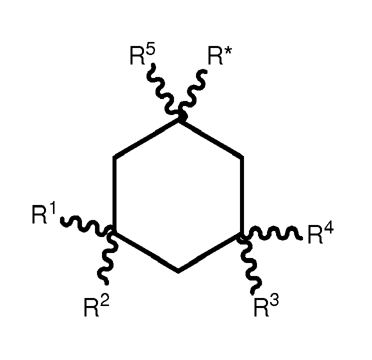 wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), and wherein R⁸ and R⁹ each represent hydrogen or lower-alkyl (1-6C) or together represent lower-alkylene -(CH₂)ₓ-wherein x is 2 to 5, inclusive, and enantiomers, optical isomers, hydrates, and pharmaceutically-acceptable salts thereof, as well as pharmaceutical compositions thereof, and the preparation and use of such compounds and compositions as 5HT₃ and neuronal nicotinic receptor antagonists and neuroprotective agents for the treatment of a living animal for the alleviation of conditions responsive thereto.

Representative of these compounds are as follows:
MRZ 2/579: 1-Amino-1,3,3,5,5-pentamethylcyclohexane, HCl
601: 1-Amino-1-propyl-3,3,5,5-tetramethylcyclohexane, HCl
607: 1-Amino-1,3,3,5(trans)-tetramethylcyclohexane (axial amino group), HCl
615: 1-Amino-1,3,5,5-tetramethyl-3-ethylcyclohexane (mixture of diastereomers), HCl
616: 1-Amino-1,3,5-trimethylcyclohexane (mixture of diastereomers), HCl
617: 1-Amino-1,3-dimethyl-3-propylcyclohexane (mixture of diastereomers), HCl
618: 1-Amino-1,3 (trans),5 (trans)-trimethyl-3(cis)-propylcyclohexane, HCl
620: 1-Amino-1,3-dimethyl-3-ethylcyclohexane, HCl
621: 1-Amino-1,3,3-trimethylcyclohexane, HCl
625: 1-Amino-1,3 (trans)-dimethylcyclohexane, HCl
627: 1-Amino-1-methyl-3 (trans) propylcyclohexane, HCl
629: 1-Amino-1-methyl-3 (trans) ethylcyclohexane, HCl
632: 1-Amino-1,3,3-trimethyl-5 (cis) ethylcyclohexane, HCl
633: 1-Amino-1,3,3-trimethyl-5 (trans) ethylcyclohexane, HCl
640: N-methyl-1-Amino-1,3,3, 5.5-pentamethylcyclohexane, HCl
641: 1-Amino-1-methylcyclohexane, HCl
642: N,N-dimethyl-1-amino-1,3,3,5,5-pentamethylcyclohexane, HCl.H₂O
705: N-(1,3,3,5,5-pentamethylcyclohexyl) pyrrolidine, HCl
680: 1-amino-1,3(trans),5(trans)-trimethylcyclohexane, HCl
681: 1-amino-1,3(cis),5(cis)-trimethylcyclohexane, HCl.H₂O,
682: 1-amino-(1R,5S)trans-5-ethyl-1,3,3-trimethylcyclohexane, HCl
683: 1-amino-(1S,5S)cis-5-ethyl-1,3,3-trimethylcyclohexane, HCl.H₂O,
1-Amino-1,5,5-trimethyl-3(cis)-isopropyl-Cyclohexane HCl,
1-Amino-1,5,5-trimethyl-3(trans)-isopropyl-cyclohexane HCl,
1-Amino-1-methyl-3 (cis) -ethyl-cyclohexane HCl,
1-Amino-1-methyl-3 (cis) -methyl-cyclohexane HCl,
1-Amino-5,5-diethyl-1,3,3-trimethyl-cyclohexane HCl, and
Also, 1-amino-1,3,3,5,5-pentamethylcyclohexane,
1-amino-1,5,5-trimethyl-3,3-diethylcyclohexane,
1-amino-l-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylCyClohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,5S)cis-5-ethyl-1,3,3-trimethylcyclohexyl] pyrrolidine or piperidine,
N-(1,3,3-trimethyl-trans-5-ethylcyclohexyl)pyrrolidine or piperidine,
N-[(1R,5S)trans-5-ethyl-1,3,3-trimethylcyclohexyl] pyrrolidine or piperidine,
N-(1-ethyl-3,3,5,5-tetramethylcyclohexyl)pyrrolidine or piperidine, and
N-(1-propyl-3,3,5,5-tetramethylcyclohexyl)pyrrolidine or piperidine,
and optical isomers, enantiomers, and the hydrochloride, hydrobromide, hydrochloride hydrate, or other pharmaceutically-acceptable salts of any of the foregoing.

Of particular interest are compounds of the foregoing formula wherein at least R¹, R⁴, and R⁵ are lower-alkyl and those compounds wherein R¹ through R⁵ are methyl, those wherein x is 4 or 5, and in particular the compound N-(1,3,3,5,5-pentamethylcyclohexyl) pyrrolidine, and optical isomers, enantiomers, hydrates and pharmaceutically-acceptable salts thereof.

In our USP 6,034,134 of March 7, 2000, we disclosed compounds of the foregoing formula, pharmaceutical compositions thereof, and their use as NMDA-receptor antagonists and anticonvulsants. It has now been found that compounds of the foregoing formula and optical isomers, enantiomers, hydrates and pharmaceutically-acceptable salts thereof, in addition to their NMDA antagonist and anticonvulsant properties, quite unpredictably possess a high degree of 5HT₃ and neuronal nicotinic receptor antagonism, making them useful in the treatment of diseases and conditions where blockade of these receptors is important.

### SUMMARY OF THE INVENTION

What we therefore believe to be comprised by our present invention may be summarized, inter alia, in the following words:

A method-of-treating a living animal for inhibition of progression or alleviation of a condition which is alleviated by a 5HT₃ or neuronal nicotinic receptor antagonist, comprising the step of administering to the said living animal an amount of a 1-aminoalkylcyclohexane compound selected from the group consisting of those of the formula 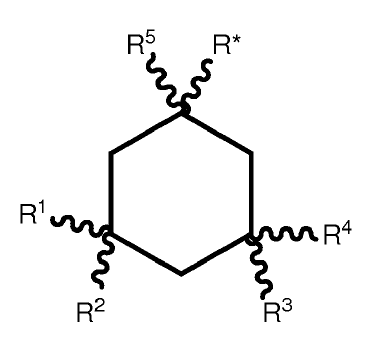 wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), wherein R⁸ and R⁹ are independently selected from the group consisting of hydrogen and lower-alkyl (1-6C) or together represent lower-alkylene -(CH₂)ₓ- wherein x is 2 to 5, inclusive, and optical isomers, enantiomers, hydrates, and pharmaceutically-acceptable salts thereof, which is effective for the said purpose; such a method wherein at least R¹, R⁴, and R⁵ are lower-alkyl; such a
method wherein R¹ through R⁵ are methyl; such a
method wherein R¹ is ethyl; such a
method wherein R² is ethyl; such a
method wherein R³ is ethyl; such a
method wherein R⁴ is ethyl; such a
method wherein R⁵ is ethyl; such a
method wherein R⁵ is propyl; such a
method wherein R⁶ or R⁷ is methyl; such a
method wherein R⁶ or R⁷ is ethyl; such a
method wherein X is 4 or 5; such a
method wherein the condition treated or inhibited is selected from the group consisting of emesis, anxiety disorders, schizophrenia, drug and alcohol abuse disorders, depressive disorders, cognitive disorders, Alzheimer's disease, cerebella tremor, Parkinson's disease, Tourette's, pain, and appetite disorders; such a
method wherein the compound is selected from the group consisting of
1-Amino-1,3,3,5,5-pentamethylcyclohexane,
1-Amino-1-propyl-3,3,5,5-tetramethylcyclohexane,
1-Amino-1,3,3,5(trans)-tetramethylcyclohexane (axial amino group),
1-Amino-1,3,5,5-tetramethyl-3-ethylcyclohexane (mixture of diastereomers),
1-Amino-1,3,5-trimethylcyclohexane (mixture of diastereomers),
1-Amino-1,3-dimethyl-3-propylcyclo-hexane (mixture of diastereomers),
1-Amino-1,3 (trans),5(trans)-trimethyl-3(cis)-propyl-cyclo-hexane,
1-Amino-1,3-dimethyl-3-ethylcyclohexane,
1-Amino-1,3,3-trimethylcyclohexane,
1-Amino-1,3(trans)-dimethylcyclohexane,
1-Amino-1-methyl-3 (trans) propylcyclohexane,
1-Amino-1-methyl-3 (trans) ethylcyclohexane,
1-Amino-1,3,3-trimethyl-5 (cis) ethylcyclohexane,
1-Amino-1,3,3-trimethyl-5 (trans) ethylcyclohexane,
N-methyl-1-Amino-1,3,3,5.5-pentamethylcyclohexane,
1-Amino-1-methylcyclohexane,
N,N-dimethyl-1-amino-1,3,3,5,5-pentamethylcyclohexane,
1-Amino-1,5,5-trimethyl-3(cis)-isopropyl-cyclohexane,
1-Amino-1,5,5-trimethyl-3(trans)-isopropyl-cyclohexane,
1-Amino-1-methyl-3(cis)-ethyl-cyclohexane,
1-Amino-1-methyl-3-(cis)-methyl-cyclohexane,
1-Amino-5,5-diethyl-1,3,3-trimethyl-cyclohexane, and
N-(1,3,3,5,5-pentamethylcyclohexyl) pyrrolidine,
and optical isomers, enantiomers, hydrates and pharmaceutically-acceptable salts of any of the foregoing; and such a

method wherein the compound is administered in the form of a pharmaceutical composition thereof comprising the compound in combination with one or more pharmaceutically-acceptable diluents, excipients, or carriers.

Moreover, a use of a 1-aminoalkylcyclohexane selected from the group consisting of those of the formula 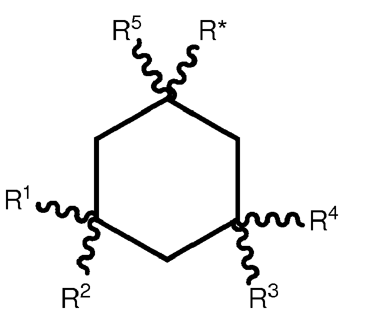 wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), wherein R⁸ and R⁹ are independently selected from the group consisting of hydrogen and lower-alkyl or together represent lower-alkylene -(CH₂)ₓ- wherein x is 2 to 5, inclusive, and optical isomers, enantiomers, hydrates, and pharmaceutically-acceptable salts thereof, in the manufacture of a medicament to treat a living animal for alleviation of a condition which is alleviated by a 5HT₃ receptor antagonist; such a
use wherein at least R¹, R⁴, and R⁵ are lower-alkyl; such a
use wherein R¹ through R⁵ are methyl; such a
use wherein x is 4 or 5; such a
use wherein the compound is selected from the group consisting of
1-Amino-1,3,3,5,5-pentamethylcyclohexane,
1-Amino-1-propyl-3,3,5,5-tetramethylcyclohexane,
1-Amino-1,3,3,5(trans)-tetramethylcyclohexane (axial amino group),
1-Amino-1,3,5,5-tetramethyl-3-ethylcyclohexane (mixture of diastereomers),
1-Amino-1,3,5-trimethylcyclohexane (mixture of diastereomers),
1-Amino-1,3-dimethyl-3-propylcyclohexane (mixture of diastereomers),
1-Amino-1,3 (trans),5 (trans)-trimethyl-3(cis)-propylcyclohexane,
1-Amino-1,3-dimethyl-3-ethylcyclohexane, 1-Amino-1,3,3-trimethylcyclohexane,
1-Amino-1,3 (trans)-dimethylcyclohexane,
1-Amino-1-methyl-3 (trans) propylcyclohexane,
1-Amino-1-methyl-3 (trans) ethylcyclohexane,
1-Amino-1,3,3-trimethyl-5 (cis) ethylcyclohexane,
1-Amino-1,3,3-trimethyl-5 (trans) ethylcyclohexane,
N-methyl-1-Amino-1,3,3,5.5-pentamethylcyclohexane,
1-Amino-1-methylcyclohexane,
N,N-dimethyl-1-amino-1,3,3,5,5-pentamethylcyclohexane,
1-Amino-1,5,5-trimethyl-3(cis)-isopropyl-cyclohexane,
1-Amino-1,5,5-trimethyl-3(trans)-isopropyl-cyclohexane,
1-Amino-1-methyl-3 (cis) -ethyl-cyclohexane,
1-Amino-1-methyl-3 (cis) -methyl-cyclohexane,
1-Amino-5,5-diethyl-1,3,3-trimethyl-cyclohexane, and
N-(1,3,3,5,5-pentamethylcyclohexyl) pyrrolidine,
and optical isomers, enantiomers, hydrates and pharmaceutically-acceptable salts of any of the foregoing; and, finally, such a
use wherein the condition treated is selected from the group consisting of emesis, anxiety disorders, schizophrenia, drug and alcohol abuse disorders, depressive disorders, cognitive disorders, Alzheimer's disease, cerebella tremor, Parkinson's disease, Tourette's, pain, and appetite disorders.

### THE PRESENT INVENTION IN DETAIL

### Background and Pharmacology

### 5-HT₃ Receptor Antagonists

5-HT₃ receptors are ligand gated ionotropic receptors permeable for cations. In man 5-HT₃ receptors show the highest density on enterochromaffin cells in the gastrointestinal mucosa, which are innervated by vagal afferents and the area postrema of the brain stem, which forms the chemoreceptor trigger zone.

Since 5-HT₃ receptors not only have a high density in the area postrema but also in the hippocampal and amygdala region of the limbic system, it has been suggested that 5-HT₃ selective antagonists may have psychotropic effects (Greenshaw & Silverstone, 1997).

Indeed, early animal studies suggested that the 5-HT₃ receptor antagonists, in addition to their well recognized anti-emetic use, may well be clinically useful in a number of areas. These include anxiety disorders, schizophrenia, drug and alcohol abuse disorders, depressive disorders, cognitive disorders, Alzheimer's disease, cerebella tremor, Parkinson's disease treatment-related psychosis, pain (migraine and irritable bowel syndrome), and appetite disorders.

### Neuronal nicotinic receptors

At present nine α subunits (α1-α2) and four β (β1-β4) subunits for nicotinic are known. α4B2 receptors are probably the most common in the CNS, especially in the hippocampus and striatum. They form non-selective cation channels with slowly, incompletely desensitizing currents (type II). Homomeric α7 receptors are both pre- and postsynaptic and are found in the hippocampus, motor cortex and limbic system as well as in the peripheral autonomic nervous system. These receptors are characterized by their high Ca²⁺ permeability and fast, strongly desensitizing responses (type 1A).

Changes in nicotinic receptors have been implicated in a number of diseases. These include Alzheimer's disease, Parkinson's disease, Tourette's, schizophrenia, drug abuse, and pain.

Based on the observation that the nicotinic agonist nicotine itself seems to have beneficial effects, drug development so far aimed at the discovery of selective nicotinic agonists.

On the other hand, it is unclear whether the effects of nicotinic agonists in, e.g., Tourette's syndrome and schizophrenia, are due to activation or inactivation / desensitization of neuronal nicotinic receptors.

The effects of agonists on neuronal nicotinic receptors is strongly dependent on the exposure period. Rapid reversible desensitization occurs in milliseconds, rundown occurs in seconds, irreversible inactivation of α4β2 and α7 containing receptors occurs in hours and their upregulation occurs within days.

In other words: the effects of nicotinic "agonists" may in fact be due to partial agonism, inactivation and/or desensitization of neuronal nicotinic receptors. In turn, moderate concentrations of neuronal nicotinic receptor channel blockers could produce the same effects as reported for nicotinic agonists in the above mentioned indications.

### Amino-alkylcyclohexanes are 5-HT3 and neuronal nicotinic receptor antagonists

We speculated whether novel amino-alkylcyclohexane derivatives (USP 6,034,134), being there described as uncompetitive NMDA receptor antagonists and anticonvulsants, might possibly also act as 5HT3 and neuronal nicotinic antagonists. These properties would allow the use of the amino-alkylcyclohexanes in all diseases or conditions where blockade of 5HT3 or nicotinic receptors is important. Our findings were positive.

### METHODS

### Synthesis

The synthesis of the novel amino-alkylcyclohexanes which are utilized according to the present invention has been described in USP 6,034,134 of March 7, 2000.

### Alternative Procedure

The 1-cyclic amino compounds may also be prepared by reacting the corresponding 1-free amino-alkylcyclohexane and the selected alpha, omega-dihaloalkyl compound, e.g., 1,3-dibromopropane, 1,4-dibromobutane, or 1,5-dibromopentane, according to the following representative example:
N-(1,3,3,5,5-pentamethylcyclohexyl)pyrrolidine hydrochloride

1,3,3,5,5-pentamethylcyclohexylamine hydrochloride (12 g, 58.3 mmol), potassium carbonate (48.4 g, 350 mmol) and 1,4-dibromobutane (7.32 ml, 61.3 mmol) were refluxed in acetonitrile (250 ml) for 60h. After cooling to r.t., the mixture was filtered and the precipitate was washed with diethyl ether (600 ml). The filtrate was concentrated in vacuo by rotary evaporation and the residue was fractionally distilled at reduced pressure (11mm/Hg). The fraction at 129°C was collected to obtain colorless oil (8.95 g). This was dissolved in diethyl ether (120 ml) and 2.7 M HCl solution in diethyl ether (30 ml) was added. The resulting precipitate was filtered off, washed with diethyl ether (3*30 ml) and dried in vacuo over NaOH to give N-(1,3,3,5,5-pentamethylcyclohexyl) pyrrolidine hydrochloride hydrate (12.9 g, 68%) with m.p. 158°C. PMR spectrum: (DMSO-d6, TMS) d: 0.97 (6H, s, 3,5-CH3); 1.11 (6H,s, 3,5-CH3); 0.8 - 1.4 (2H, cyclohexane 4-CH2) 1.41 (3H, s, 1-CH3); 1.69 (4H, m, cyclohexane 2,6-CH2); 1.84 (4H, m, pyrrolidine 3,4-CH2); 3.20 (4H, m, pyrrolidine 2,5-CH2); 10.9 ppm (1H, br s, NH+).

*Elemental analysis* (C15H29n*HCl*H2O) Found (%) C 65.0; H 11.7; N5.0 Calculated (%) C 64.8; H 11.6; N 5.0.

### Electrophysiology

Hippocampi were obtained from rat embryos (E20 to E21) and were then transferred to Ca²⁺ and Mg²⁺ free Hank's buffered salt solution (Gibco) on ice. Cells were mechanically dissociated in 0.05% DNAase / 0.3% ovomucoid (Sigma) following an 8 minute pre-incubation with 0.66% trypsin / 0.1% DNAase (Sigma). The dissociated cells were then centrifuged at 18G for 10 minutes, re-suspended in minimum essential medium (Gibco) and plated at a density of 150,000 cells cm² onto poly-DL-ornithine (Sigma) / laminin (Gibco) - precoated plastic Petri dishes (Falcon). The cells were nourished with NaHCO₃/HEPES-buffered minimum essential medium supplemented with 5% foetal calf serum and 5% horse serum (Gibco) and incubated at 37°C with 5%CO₂ at 95% humidity. The medium was exchanged completely following inhibition of further glial mitosis with cytosine-β-D-arabinofuranoside (ARAC, 5 µM Sigma) after about 5 days *in vitro.*

Patch clamp recordings were made from these neurones after 15-21 days *in vitro* with polished glass electrodes (2-3 MΩ) in the whole cell mode at room temperature (20-22°C) with the aid of an EPC-7 amplifier (List). Test substances were applied using a modified fast application system (SF-77B Fast Step, Warner Instruments) with 100 µM opening diameter theta glass (Clark TGC 200-10) pulled with a Zeiss DMZ (Augsburg, Munich) horizontal puller. The contents of the intracellular solution were normally as follows (mM): CsCl (95), TEACl (20), EGTA (10), HEPES (10), MgCl₂ (1), CaCl₂ (0.2), glucose (10), Tris-ATP (5), Di-Tris-Phosphocreatinine (20), Creatine Phosphokinase (50 U); pH was adjusted to 7.3 with CsOH or HCl. The extracellular solutions had the following basic composition (mM): NaCl (140), KCl (3), CaCl₂ (0.2), glucose (10), HEPES (10), sucrose (4.5), tetrodotoxin (TTX 3*10⁴).

N1E-115 cells were purchased from the European collection of cell cultures (ECACC, Salisbury, UK) and stored at -80°C until further use. The cells were plated at a density of 100,000 cells cm⁻² onto plastic Petri dishes (Falcon) and were nourished with NaHCO₃/HEPES-buffered minimum essential medium (MEM) supplemented with 15% foetal calf serum (Gibco) and incubated at 37°C with 5%CO₂ at 95% humidity. The medium was exchanged completely daily. Once every three days, cells were re-seeded onto fresh Petri dishes following treatment with trypsin-EDTA (1% in PBS), resuspension in MEM, and centrifugation at 1000 for 4 mins.

Patch clamp recordings were made from lifted cells, 2-3 days following seeding with polished glass electrodes (2-3 MΩ) in the whole cell mode at room temperature (20-22°C) with an EPC-7 amplifier (List). Test substances were applied as for hippocampal cells. The contents of the intracellular solution were as follows (mM): CsCl (130), HEPES (10), EGTA (10), MgCl₂ (2), CaCl₂ (2), K-ATP (2), Tris-GTP (0.2), D-Glucose (10); pH was adjusted to 7.3 with CsOH or HCl. The extracellular solutions had the following basic composition (mM): NaCl (124), KCl (2.8), HEPES (10), pH 7.3 with NaOH or HCl.

Only results from stable cells were accepted for inclusion in the final analysis, i.e., showing at least 75% recovery of responses to agonist (serotonin or Ach) following removal of the antagonist tested. Despite this, recovery from drug actions wasn't always 100% because of rundown in some cells (<= 10% over 10 mins). When present, this was always compensated by basing the % antagonism at each concentration on both control and recovery and assuming a linear time course for this rundown. All antagonists were assessed at steady-state blockade with 3 to 6 concentrations on at least 5 cells. Equilibrium blockade was achieved within 2 to 5 agonist applications, depending on antagonist concentration.

### Results

Table 1 shows the general structure of selected amino-alkylcyclohexanes used in the present study.

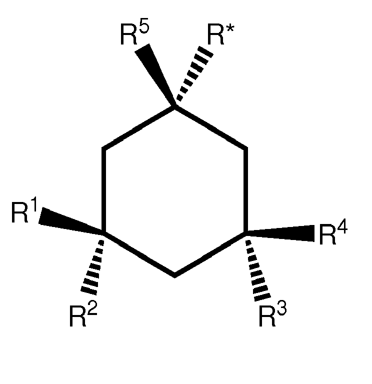

**Table 1**

| **Basic Structure of the Amino-alkylcyclohexanes** | | | | | | |
|---|---|---|---|---|---|---|
| MRZ | R1 | R2 | R3 | R4 | R5 | R* |
| 579 | CH₃ | CH₃ | CH₃ | CH₃ | CH₃ | NH₂ |
| 601 | CH₃ | CH₃ | CH₃ | CH₃ | C₃H₇ | NH₂ |
| 607 | CH₃ | CH₃ | H | CH₃ | C₃H₇ | NH₂ |
| 615 | CH₃ | CH₃ | C₂H₅(CH₃) | CH₃(C₂H₅) | CH₃ | NH₂ |
| 616 | CH₃(H) | H(CH₃) | H(CH₃) | CH₃(H) | CH₃ | NH₂ |
| 617 | H | H | CH₃(C₃H₇) | C₃H₇(CH₃) | CH₃ | NH₂ |
| 618 | CH₃ | H | C₃H₇ | CH₃ | CH₃ | NH₂ |
| 620 | H | H | C₂H₅(CH₃) | CH₃(C₂H₅) | CH₃ | NH₂ |
| 621 | H | H | CH₃ | CH₃ | CH₃ | NH₂ |
| 625 | H | H | H | CH₃ | CH₃ | NH₂ |
| 627 | H | H | H | C₃H₇ | CH₃ | NH₂ |
| 629 | H | H | H | C₂H₅ | CH₃ | NH₂ |
| 632 | CH₃ | CH₃ | C₂H₅ | H | CH₃ | NH₂ |
| 633 | CH₃ | CH₃ | H | C₂H₅ | CH₃ | NH₂ |
| 640 | CH₃ | CH₃ | CH₃ | CH₃ | CH₃ | NHCH₃ |
| 641 | H | H | H | H | CH₃ | NH₂ |
| 642 | CH₃ | CH₃ | CH₃ | CH₃ | CH₃ | NH(CH₃)₂ |
| 705 | CH₃ | CH₃ | CH₃ | CH₃ | CH₃ | NH(CH₂)₄ |

Substitutions in brackets represent alternatives in racemic mixtures, e.g., CH₃(C₃H₇) means CH₃ or C₃H₇.

### BRIEF DESCRIPTION OF THE DRAWINGS:

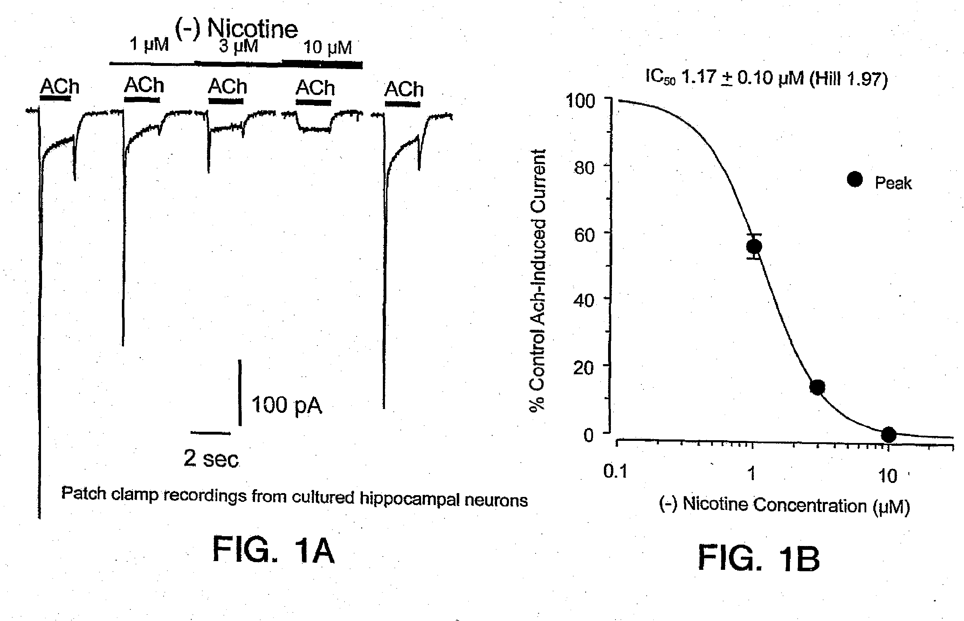

FIG. 1A and FIG. 1B show concentration-dependence of the blockade of 5HT3 receptors by MRZ 2/633 in cultured N1E-115 cells. Serotonin (10µM) was applied for 2 seconds every 30 seconds in the continuous presence of various concentrations of MRZ 2/633 (1-10µM).
   A: Original data for a single N1E-115 cell - serotonin was applied as indicated by the bars. The left and right panels show control and recovery responses respectively. The middle three panels show equilibrium responses in the continuous presence of MRZ 2/633 1, 3, and 10 µM respectively.
   B: Peak and steady-state (plateau) serotonin current responses were normalized to control levels and plotted as means (±SEM) against MRZ 2/633 concentration (n=8). Estimation of IC₅₀s and curve fitting were made according to the 4 parameter logistic equation (GraFit, Erithacus Software).

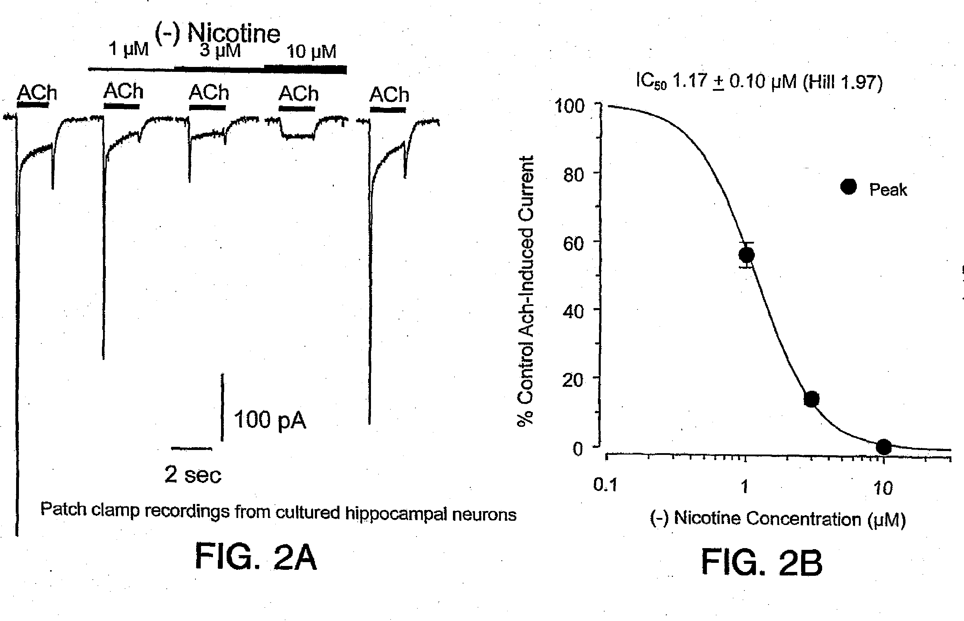

FIG. 2A and FIG. 2B show that nicotine acts as a functional antagonist of neuronal nicotinic (type Ia = α7) receptors in hippocampal neurones by inducing receptor desensitization. Ach (1 mM) was applied for 2 seconds every 30 seconds in the continuous presence of various concentrations of (-) nicotine (1-10µM).
   A: Original data for a single hippocampal neurone - Ach was applied as indicated by the bars. The left and right panels show control and recovery responses respectively. The middle three panels show equilibrium responses in the continuous presence of (-)nicotine 1, 3 and 10 µM respectively.
   B: Peak ACh current responses were normalized to control levels and plotted as means (±SEM) against (-) nicotine concentration (n=12 per concentration). Estimation of IC₅₀s and curve fitting were made according to the 4 parameter logistic equation (GraFit, Erithacus Software).

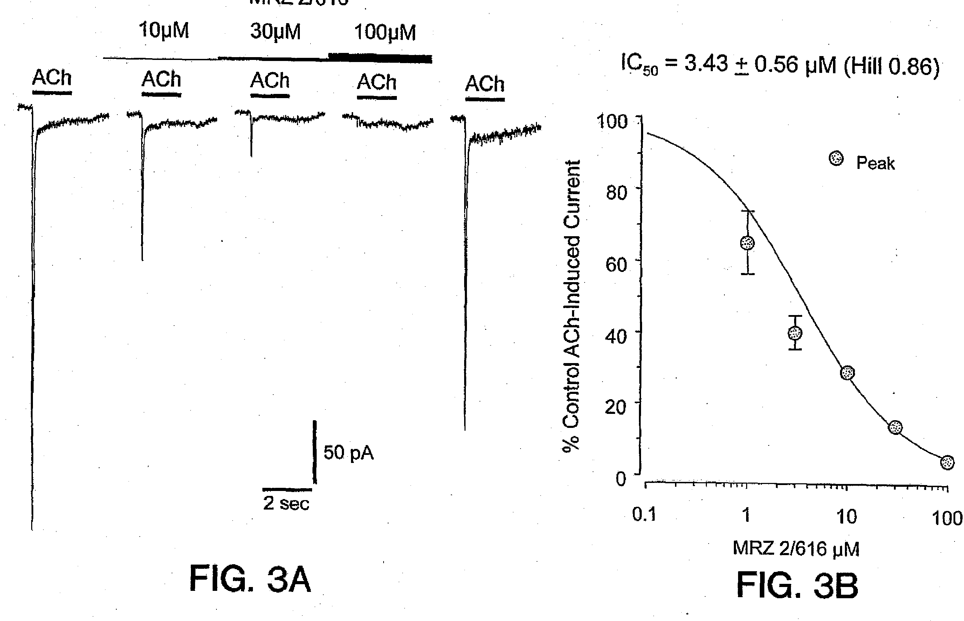

FIG. 3A and FIG. 3B show a concentration-dependence of the blockade of neuronal nicotinic (type Ia = α7) receptors by MRZ 2/616 in hippocampal neurones. Ach (1 mM) was applied for 2 seconds every 30 seconds in the continuous presence of various concentrations of MRZ 2/616 (1-100µM).
   A: Original data for a single hippocampal neurone - Ach was applied as indicated by the bars. The left and right panels show control and recovery responses respectively. The middle three panels show equilibrium responses in the continuous presence of MRZ 2/616 10, 30 and 100 µM respectively
   B: Peak ACh current responses were normalized to control levels and plotted as means (±SEM) against MRZ 2/616 concentration (n=11 per concentration). Estimation of IC₅₀s and curve fitting were made according to the 4 parameter logistic equation (GraFit, Erithacus Software).

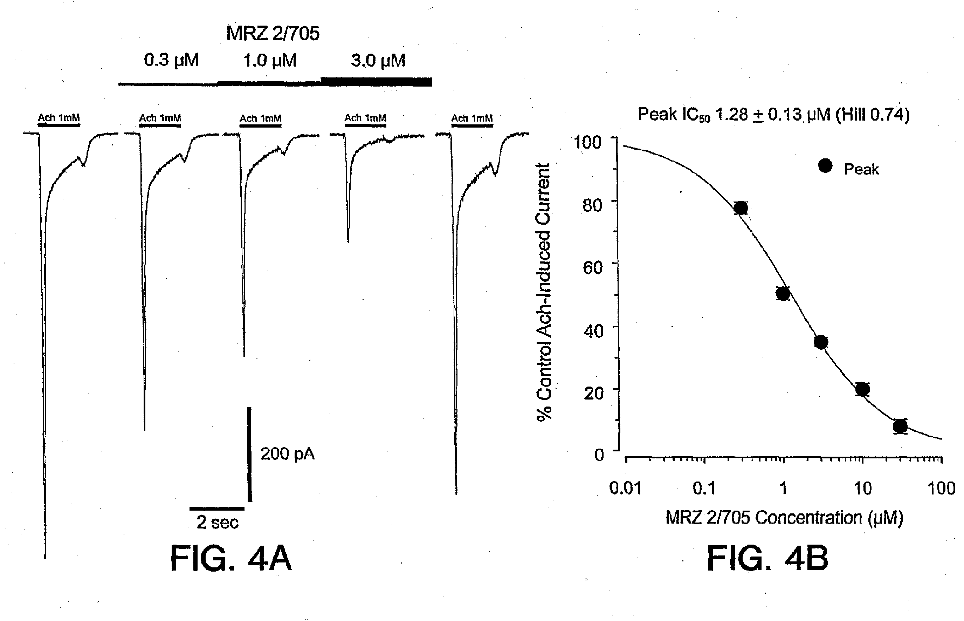

FIG. 4A and FIG. 4B show concentration-dependence of the blockade of neuronal nicotinic (type Ia = α7) receptors by MRZ 2/705 in hippocampal neurones. Ach (1 mM) was applied for 2 seconds every 30 seconds in the continuous presence of various concentrations of MRZ 2/705 (0.3-30µM).
   A: Original data for a single hippocampal neurone - Ach was applied as indicated by the bars. The left and right panels show control and recovery responses respectively. The middle three panels show equilibrium responses in the continuous presence of MRZ 2/705 0.3, 1.0 and 3.0 µM respectively
   B: Peak ACh current responses were normalized to control levels and plotted as means (±SEM) against MRZ 2/705 concentration (n=9 per concentration). Estimation of IC50s and curve fitting were made according to the 4 parameter logistic equation (GraFit, Erithacus Software).

### Effects of amino-alkylcyclohexanes on 5-HT₃ receptors

All ten amino-alkylcyclohexanes tested antagonized serotonin-induced inward currents in N1E-115 cells with similar potencies to those previously reported for NMDA-induced inward currents (Fig. 1, see also Parsons et al., 1999). Similar effects were seen with the same compounds when tested on 5-HT₃ receptors permanently expressed in HEK-293 cells. As such, the amino-alkylcyclohexanes tested had similar effects on 5-HT₃ receptors as those previously reported for a variety of anti-depressants (Fan, 1994), i.e., they antagonized responses by inducing desensitization.

**Table 2**

| MRZ 2/ | [³H]MK- | PC NMDA | 5HT₃ |
|---|---|---|---|
| 579 | 1.4 | 1.3 | 1.7 |
| 601 | 7.7 | 10.0 | 1.3 |
| 607 | 7.7 | 13.8 | 22.3 |
| 615 | 2.29 | 1.30 | 2.5 |
| 616 | 10.4 | 33.2 | 38.7 |
| 621 | 30.6 | 92.4 | 20.3 |
| 632 | 2.8 | 6.4 | 2.4 |
| 633 | 4.7 | 13.9 | 7.7 |
| 640 | 4.8 | 14.6 | 10.8 |
| 642 | 10.7 | 42.5 | 35.5 |

Summary of the potencies of amino-alkylcyclohexanes on NMDA and 5-HT₃ receptors. Data for displacement of [³H]MK-801 binding in rat cortical membranes and antagonism of NMDA-induced inward currents (at -70mV) in cultured rat hippocampal neurones are taken from Parsons et al., 1999. Potencies against 5-HT₃ receptors were assessed as IC₅₀s (µM) against "steady-state" responses of N1E-115 cells to serotonin (10µM) applied for 2 secs.

### Effects of amino-alkylcyclohexanes on neuronal nicotinic receptors

Concentration-clamp application of Ach (1mM) to cultured hippocampal neurones elicited rapid, pronounced inward currents which rapidly desensitized to a much lower plateau level. Nicotine caused a concentration dependent block of neuronal responses to Ach and concentrations achieved in the CNS of smokers caused a substantial antagonism (Fig. 2, IC₅₀ = 1.17 µM).

We next accessed the potencies of a variety of amino-alkylcyclohexanes as α7 neuronal nicotinic antagonists. Simple amino-alkylcyclohexanes with low alkyl substitutions at positions R1 through R4 (see Table 1) were potent α7 neuronal nicotinic antagonists and some, as exemplified by MRZ 2/616 were actually much more potent in this regard than previously reported for NMDA receptors (see Fig. 3 and Parsons et al., 1999). The N-pyrollidine derivative MRZ 2/705 was also 16 fold more effective as an α7 neuronal nicotinic antagonist than as an NMDA receptor antagonist (Table 3 and Fig. 4).

**Table 3**

| MRZ | [³H]MK | PC | PC Ach |
|---|---|---|---|
| 579 | 1.44 | 1.30 | 30.00 |
| 615 | 2.29 | 2.90 | 2.21 |
| 616 | 9.94 | 33.20 | 3.40 |
| 617 | 36.08 | 63.90 | 1.16 |
| 618 | 22.79 | 57.50 | 0.65 |
| 620 | 24.18 | 99.00 | 2.44 |
| 621 | 30.56 | 92.40 | 0.65 |
| 625 | 48.98 | 244.90 | 3.29 |
| 627 | 67.30 | 150.00 | 2.60 |
| 629 | 46.74 | 218.60 | 2.05 |
| 641 | 135.86 | >100 | 2.40 |
| 642 | 10.73 | 42.50 | 1.00 |
| 705 | 7.09 | 20.80 | 1.30 |

Summary of the potencies of amino-alkylcyclohexanes on NMDA and α7 neuronal nicotinic receptors. Data for displacement of [³H]MK-801 binding in rat cortical membranes and antagonism of NMDA-induced inward currents (at -70mV, PC NMDA) in cultured rat hippocampal neurones are taken from Parsons et al., 1999. Potencies against α7 neuronal nicotinic receptors (PC ACh) were assessed as IC₅₀s (µM) against peak responses of cultured hippocampal neurones to ACh (1 mM) applied for 2 secs.

### Conclusions

The present data show that amino-alkylcyclohexanes are antagonists of 5-HT₃ receptors. These effects were seen at concentrations similar to, or even lower than, those required for uncompetitive antagonistic effects at NMDA receptors as reported by Parsons et al. 1999. Combined antagonistic effects of such compounds at NMDA and 5-HT₃ receptors will therefore lead to positive synergistic effects contributing to their therapeutic safety and efficacy in Alzheimer's disease by increasing desired effects - cognitive enhancement and antidepressant effects - whilst further reducing possible negative effects of NMDA receptor antagonism by, e.g., reducing mesolimbic dopamine hyperactivity. Furthermore, 5-HT₃ antagonistic effects *per se* are useful in the treatment of cognitive deficits, depression, alcohol abuse, anxiety, migraine, irritable bowel syndrome, and emesis.

The present data show also that some amino-alkylcyclohexanes are in fact more potent as α7 neuronal nicotinic receptor antagonists than for actions at NMDA and/or 5-HT₃ receptors. It is likely that many of these agents are also antagonists of α4ß2 receptors, as already reported for agents like memantine and amantadine by Buisson et al. (1998). We propose that the positive effects reported by others for neuronal nicotinic agonists in animal models of various diseases are actually due to desensitization of α7 receptors and inactivation / down regulation of α4/ß2 receptors or other forms of functional antagonism by, e.g., partial agonistic effects. Moderate concentrations of neuronal nicotinic receptor antagonists are therefore useful for neuroprotection against, or for the treatment of, disorders related to the malfunction of nicotinic transmission such as, e.g., Alzheimer's disease, Parkinson's disease, schizophrenia, Tourette's syndrome, drug abuse, and pain.

### PHARMACEUTICAL COMPOSITIONS

The active ingredients of the invention, together with one or more conventional adjuvants, carriers, or diluents, may be placed into the form of pharmaceutical compositions and unit dosages thereof, and in such form may be employed as solids, such as coated or uncoated tablets or filled capsules, or liquids, such as solutions, suspensions, emulsions, elixirs, or capsules filled with the same, all for oral use; in the form of suppositories or capsules for rectal administration or in the form of sterile injectable solutions for parenteral (including intravenous or subcutaneous) use. Such pharmaceutical compositions and unit dosage forms thereof may comprise conventional or new ingredients in conventional or special proportions, with or without additional active compounds or principles, and such unit dosage forms may contain any suitable effective amount of the active ingredient commensurate with the intended daily dosage range to be employed. Tablets containing twenty (20) to one hundred (100) milligrams of active ingredient or, more broadly, ten (10) to two hundred fifty (250) milligrams per tablet, are accordingly representative unit dosage forms.

### METHOD OF TREATING

Due to their high degree of activity and their low toxicity, together presenting a most favorable therapeutic index, the active principles of the invention may be administered to a subject, e.g., a living animal (including a human) body, in need thereof, for the treatment, alleviation, or amelioration, palliation, or elimination of an indication or condition which is susceptible thereto, or representatively of an indication or condition set forth elsewhere in this application, preferably concurrently, simultaneously, or together with one or more pharmaceutically-acceptable excipients, carriers, or diluents, especially and preferably in the form of a pharmaceutical composition thereof, whether by oral, rectal, or parental (including intravenous and subcutaneous) or in some cases even topical route, in an effective amount. Dosage ranges may be 1-1000 milligrams daily, preferably 10-500 milligrams daily, and especially 50-500 milligrams daily, depending as usual upon the exact mode of administration, form in which administered, the indication toward which the administration is directed, the subject involved and the body weight of the subject involved, and the preference and experience of the physician or veterinarian in charge.

### EXAMPLES OF REPRESENTATIVE PHARMACEUTICAL COMPOSITIONS

With the aid of commonly used solvents, auxiliary agents and carriers, the reaction products can be processed into tablets, coated tablets, capsules, drip solutions, suppositories, injection and infusion preparations, and the like and can be therapeutically applied by the oral, rectal, parenteral, and additional routes. Representative pharmaceutical compositions follow.
(a) Tablets suitable for oral administration which contain the active ingredient may be prepared by conventional tabletting techniques.
(b) For suppositories, any usual suppository base may be employed for incorporation thereinto by usual procedure of the active ingredient, such as a polyethyleneglycol which is a solid at normal room temperature but which melts at or about body temperature.
(c) For parental (including intravenous and subcutaneous) sterile solutions, the active ingredient together with conventional ingredients in usual amounts are employed, such as for example sodium chloride and double-distilled water q.s., according to conventional procedure, such as filtration, aseptic filling into ampoules or IV-drip bottles, and autoclaving for sterility.

Other suitable pharmaceutical compositions will be immediately apparent to one skilled in the art.

The following examples are given by way of illustration only and are not to be construed as limiting.

### EXAMPLE 1

### Tablet Formulation

A suitable formulation for a tablet containing 10 milligrams of active ingredient is as follows:

| | Mg. |
|---|---|
| Active Ingredient | 10 |
| Lactose | 63 |
| Microcrystalline | |
| Cellulose | 21 |
| Talcum | 4 |
| Magnesium stearate | 1 |
| Colloidal silicon | |
| dioxide | 1 |

### EXAMPLE 2

### Tablet Formulation

Another suitable formulation for a tablet containing 100 mg is as follows:

| | Mg. |
|---|---|
| Active Ingredient | 100 |
| Potato starch | 20 |
| Polyvinylpyrrolidone | 10 |
| Film coated and colored. | |
| The film coating material consists of: | |
| Lactose | 100 |
| Microcryst. Cellulose | 80 |
| Gelatin | 10 |
| Polyvinylpyrrolidone, crosslinked | 10 |
| Talcum | 10 |
| Magnesium stearate | 2 |
| Colloidal silicon dioxide | 3 |
| Color pigments | 5 |

### EXAMPLE 3

### Capsule Formulation

A suitable formulation for a capsule containing 50 milligrams of active ingredient is as follows:

| | Mg. |
|---|---|
| Active Ingredient | 50 |
| Corn starch | 20 |
| | |
| Dibasic calcium phosphate | 50 |
| Talcum | 2 |
| Colloidal silicon dioxide | 2 |

filled in a gelatin capsule.

### EXAMPLE 4

### Solution for injection

A suitable formulation for an injectable solution containing one percent of active ingredient is as follows:

| | | |
|---|---|---|
| Active Ingredient | mg | 12 |
| Sodium chloride | mg | 8 |
| Sterile water to make | ml | 1 |

### EXAMPLE 5

### Liquid oral formulation

A suitable formulation for 1 liter of a liquid mixture containing 2 milligrams of active ingredient in one milliliter of the mixture is as follows:

| | G. |
|---|---|
| Active Ingredient | 2 |
| Saccharose | 250 |
| Glucose | 300 |
| Sorbitol | 150 |
| Orange flavor | 10 |
| Sunset yellow. | |
| Purified water to make a total of 1000 ml. | |

### EXAMPLE 6

### Liquid oral formulation

Another suitable formulation for 1 liter of a liquid mixture containing 20 milligrams of active ingredient in one milliliter of the mixture is as follows:

| | G. |
|---|---|
| Active Ingredient | 20 |
| Tragacanth | 7 |
| Glycerol | 50 |
| Saccharose | 400 |
| Methylparaben | 0.5 |
| Propylparaben | 0.05 |
| Black currant-flavor | 10 |
| Soluble Red color | 0.02 |
| Purified water to make a total of 1000 ml. | |

### EXAMPLE 7

### Liquid oral formulation

Another suitable formulation for 1 liter of a liquid mixture containing 2 milligrams of active ingredient in one milliliter of the mixture is as follows:

| | G. |
|---|---|
| Active Ingredient | 2 |
| Saccharose | 400 |
| Bitter orange peel tincture | 20 |
| Sweet orange peel tincture | 15 |
| Purified water to make a total of 1000 ml. | |

### EXAMPLE 8

### Aerosol formulation

180 g aerosol solution contain:

| | G. |
|---|---|
| Active Ingredient | 10 |
| Oleic acid | 5 |
| Ethanol | 81 |
| Purified Water | 9 |
| Tetrafluoroethane | 75 |

15 ml of the solution are filled into aluminum aerosol cans, capped with a dosing valve, purged with 3.0 bar.

### EXAMPLE 9

### TDS formulation

100 g solution contain:

| | G. |
|---|---|
| Active Ingredient | 10.0 |
| Ethanol | 57.5 |
| Propyleneglycol | 7.5 |
| Dimethylsulfoxide | 5.0 |
| Hydroxyethylcellulose | 0.4 |
| Purified water | 19.6 |

1.8 ml of the solution are placed on a fleece covered by an adhesive backing foil. The system is closed by a protective liner which will be removed before use.

### EXAMPLE 10

### Nanoparticle formulation

10 g of polybutylcyanoacrylate nanoparticles contain:

| | G. |
|---|---|
| Active Ingredient | 1.0 |
| Poloxamer | 0.1 |
| Butylcyanoacrylate | 8.75 |
| Mannitol | 0.1 |
| Sodiumchloride | 0.05 |

Polybutylcyanoacrylate nanoparticles are prepared by emulsion polymerization in a water/0.1 N HCl/ethanol mixture as polymerization medium. The nanoparticles in the suspension are finally lyophilized under vacuum.

The compounds of the invention thus find application in the treatment of disorders of a living animal body, especially a human, in both 5HT₃ and nicotinic receptor indications for both symptomatic and neuroprotective purposes

The method-of-treating a living animal body with a compound of the invention, for the inhibition of progression or alleviation of the selected ailment therein, is as previously stated by any normally-accepted pharmaceutical route, employing the selected dosage which is effective in the alleviation of the particular ailment desired to be alleviated.

Use of the compounds of the present invention in the manufacture of a medicament for the treatment of a living animal for inhibition of progression or alleviation of the selected ailment or condition, particularly ailments or conditions susceptible to treatment with a 5HT₃ or nicotinic receptor antagonist, is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament are all in accord with the foregoing and with the disclosure of our prior USP 6,034,134 for the same 1-amino compounds, and representative acid addition salts, enantiomers, isomers, and hydrates, and their method of preparation is likewise disclosed in our prior USP and published WO application for the 1-amino-alkylcyclohexane compounds.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the foregoing and also in accord with examples of pharmaceutical compositions given in our U.S. patent 6,034,134 for these 1-amino-alkylcyclohexanes.

It is to be understood that the invention is not to be limited to the exact details of operation, or to the exact compositions, methods, procedures, or embodiments shown and described, as obvious modifications and equivalents will be apparent to one skilled in the art, and the invention is therefore to be limited only by the full scope which can be legally accorded to the appended claims.

### REFERENCES

Buisson, B., Bertrand, D., 1998, Open-channel blockers at the human alpha4beta2 neuronal nicotinic acetylcholine receptor. Mol. Pharmacol. 53, 555-563.
Fan, P., 1994, Effects of antidepressants on the inward current mediated by 5-HT3 receptors in rat nodose ganglion neurones. Br J Pharmacol 112, 741-744.
Greenshaw, A.J., Silverstone, P.H., 1997, The non-antiemetic uses of serotonin 5-HT3 receptor antagonists. Clinical pharmacology and therapeutic applications. Drugs 53, 20-39.
Parsons, C.G., Danysz, W., Bartmann, A., Spielmanns, P., Frankiewicz, T., Hesselink, M., Eilbacher, B., Quack, G., 1999, Amino-alkylcyclohexanes are novel uncompetitive NMDA receptor antagonists with strong voltage-dependency and fast blocking kinetics: in vitro and in vivo characterization. Neuropharmacology 38, 85-108.

Key embodiments of the invention include the following:

In a first embodiment, the invention comprises a method-of-treating a living animal for inhibition of progression or alleviation of a condition which is alleviated by a 5HT₃ or neuronal nicotinic receptor antagonist, comprising the step of administering to the said living animal an amount of a 1-aminoalkylcyclohexane compound selected from the group consisting of those of the formula 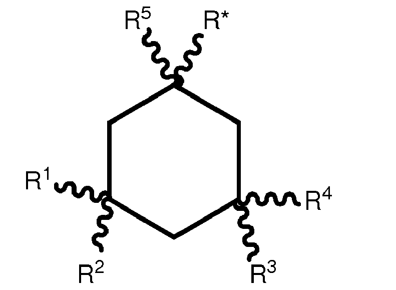 wherein R* is -(CH₂)ₙ-(CR⁶R⁷)ₘ-NR⁸R⁹
wherein n+m = 0, 1, or 2
wherein R¹ through R⁷ are independently selected from the group consisting of hydrogen and lower-alkyl (1-6C), wherein R⁸ and R⁹ are independently selected from the group consisting of hydrogen and lower-alkyl (1-6C) or together represent lower-alkylene -(CH₂)ₓ- wherein x is 2 to 5, inclusive, and optical isomers, enantiomers, hydrates, and pharmaceutically-acceptable salts thereof, which is effective for the said purpose.

In a second embodiment, the invention comprises the method of the first embodiment, wherein at least R¹, R⁴, and R⁵ are lower-alkyl.

In a third embodiment, the invention comprises the method of the second embodiment, wherein R¹ through R⁵ are methyl.

In a fourth embodiment, the invention comprises the method of the first embodiment, wherein on of R¹, R², R³, R⁴, R⁵ , R⁶ and R⁷ is ethyl.

In a fifth embodiment, the invention comprises the method of the first embodiment, wherein R⁵ is propyl.

In a sixth embodiment, the invention comprises the method of any of the first to third embodiments, wherein R⁶ or R⁷ is methyl.

In a seventh embodiment, the invention comprises the method of any of the second to sixth embodiments, wherein R⁸ and R⁸ together represent a C₄ or C₅ alkylene group.

In an eighth embodiment, the invention comprises the method of the first embodiment, wherein the compound is selected from
1-Amino-1,3,3,5,5-pentamethylcyclohexane,
1-Amino-1-propyl-3,3,5,5-tetramethylcyclohexane,
1-Amino-1,3,3,5(trans)-tetramethylcyclohexane (axial amino group),
1-Amino-1,3,5,5-tetramethyl-3-ethylcyclohexane (mixture of diastereomers),
1-Amino-1,3,5-trimethylcyclohexane (mixture of diastereomers),
1-Amino-1,3-dimethyl-3-propylcyclohexane (mixture of diastereomers),
1-Amino-1,3 (trans),5 (trans)-trimethyl-3(cis)-propylcyclohexane,
1-Amino-1,3-dimethyl-3-ethylcyclohexane,
1-Amino-1,3,3-trimethylcyclohexane,
1-Amino-1,3 (trans)-dimethylcyclohexane,
1-Amino-1-methyl-3 (trans) propylcyclohexane,
1-Amino-1-methyl-3 (trans) ethylcyclohexane,
1-Amino-1,3,3-trimethyl-5 (cis) ethylcyclohexane,
1-Amino-1,3,3-trimethyl-5 (trans) ethylcyclohexane,
N-methyl-1-Amino-1,3,3,5.5-pentamethylCyClohexane,
1-Amino-1-methylcyclohexane,
N,N-dimethyl-1-amino-1,3,3,5,5-pentamethylcyclohexane,
1-Amino-1,5,5-trimethyl-3(cis)-isopropyl-cyclohexane,
1-Amino-1,5,5-trimethyl-3(trans)-isopropyl-cyclohexane,
1-Amino-1-methyl-3(cis)-ethyl-cyclohexane,
1-Amino-1-methyl-3(cis)-methyl-cyclohexane,
1-Amino-5,5-diethyl-1,3,3-trimethyl-cyclohexane, and N-(1,3,3,5,5-pentamethylcyclohexyl) pyrrolidine,
and optical isomers, enantiomers, hydrates and pharmaceutically-acceptable salts of any of the foregoing.

In a ninth embodiment, the invention comprises the method of any of the first to eighth embodiments, wherein the condition alleviated or inhibited is selected from emesis, anxiety disorders, schizophrenia, drug and alcohol abuse disorders, depressive disorders, cognitive disorders, Alzheimer's disease, cerebella tremor, Parkinson's disease, Tourette's syndrome, pain, and appetite disorders.

In a tenth embodiment, the invention comprises the method of any of the first to ninth embodiments, wherein the compound is administered in the form of a pharmaceutical composition thereof comprising the compound in combination with one or more pharmaceutically-acceptable diluents, excipients, or carriers.

In an eleventh embodiment, the invention comprises the use of the 1-aminoalkylcyclohexane of any of the first to eighth embodiments, including the optical isomers, enantiomers, hydrates, and pharmaceutically-acceptable salts thereof, in the manufacture of a medicament to treat a living animal for inhibition of progression or alleviation of a condition which is alleviated by a 5HT₃ or neuronal nicotinic receptor antagonist.

In an twelfth embodiment, the invention comprises the use of the 1-aminoalkylcyclohexane of any of the first to eighth embodiments, including the optical isomers, enantiomers, hydrates, and pharmaceutically-acceptable salts thereof, for use in treating a condition which is alleviated by a 5HT₃ or neuronal nicotinic receptor antagonist.

In an thirteenth embodiment, the invention comprises the use of either of the eleventh or twelfth embodiments, wherein the condition alleviated or inhibited is selected from the group consisting of emesis, anxiety disorders, schizophrenia, drug and alcohol abuse disorders, depressive disorders, cognitive disorders, Alzheimer's disease, cerebella tremor, Parkinson's disease, Tourette's syndrome, pain, and appetite disorders.

## Claims

1. A 1-aminoalkylcyclohexane compound of formula (I) 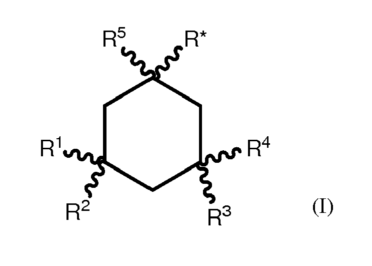 wherein:
R* is -(CH₂)ₙ-(CR⁶R⁷)ₘ-NR⁸R⁹;
n+m = 0, 1, or 2;
R¹ through R⁷ are independently selected from hydrogen and C₁₋₆ alkyl;
R⁸ and R⁹ are independently selected from hydrogen and
C₁₋₆ alkyl or together represent a C₂₋₅-alkylene group;
or an optical isomer, enantiomer, hydrate or pharmaceutically acceptable salt thereof, for use in the inhibition of progression of or for alleviation of a condition selected from irritable bowel syndrome, cerebella tumour, cognitive disorders and migraine.

2. A compound as claimed in claim 1 wherein at least R¹, R⁴ and R⁵ in formula (I) are C₁₋₆ alkyl.

3. A compound as claimed in claim 2 wherein R¹ through R⁵ in formula (I) are methyl.

4. A compound as claimed in claim 1 wherein one of R¹, R², R³, R⁴, R⁵, R⁶ and R⁷ in formula (I) is ethyl.

5. A compound as claimed in claim 1 wherein R⁵ in formula (I) is propyl.

6. A compound as claimed in any one of claims 1 to 3 wherein R⁶ or R⁷ in formula (I) is methyl.

7. A compound as claimed in any one of claims 2 to 6 wherein R⁸ and R⁹ in formula (I) together represent a C₄ or C₅ alkylene group.

8. A compound as claimed in claim 1 wherein the 1-aminoalkylcyclohexane is:
1-amino-1,3,3,5,5-pentamethylcyclohexane,
1-amino-1-propyl-3,3,5,5-tetramethylcyclohexane,
1-amino-1,3,3,5(trans)-tetramethylcyclohexane (axial amino group),
1-amino-1,3,5,5-tetramethyl-3-ethylcyclohexane (mixture of diastereomers),
1-amino-1,3,5-trimethylcyclohexane (mixture of diastereomers),
1-amino-1,3-dimethyl-3-propylcyclohexane (mixture of diastereomers),
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
1-amino-1,3(trans)-dimethylCyClohexane,
1-amino-1-methyl-3(trans)-propylcyclohexane,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
N-methyl-1-amino-1,3,3,5,5-pentamethylcyclohexane,
1-amino-1-methylcyclohexane,
N,N-dimethyl-l-amino-1,3,3,5,5-pentamethylcyclohexane
1-amino-1,5,5-trimethyl-3(cis)-isopropylcyclohexane,
1-amino-1,5,5-trimethyl-3(trans)-isopropylcyclohexane,
1-amino-1-methyl-3(cis)-ethylcyclohexane,
1-amino-1-methyl-3(cis)-methylcyclohexane,
1-amino-5,5-diethyl-1,3,3-trimethylcyclohexane,
N-(1,3,3,5,5-pentamethylcyclohexyl)pyrrolidine and optical isomers, enantiomers, hydrates and pharmaceutically acceptable salts of any of the foregoing.
